# EUROPÄISCHE PATENTSCHRIFT

(11) **EP 1 649 272 B1**
(45) Veröffentlichungstag und Bekanntmachung des Hinweises auf die Patenterteilung: **25.01.2012**
(21) Anmeldenummer: 04763472.0
(22) Anmeldetag: 24.07.2004
(51) Int. Cl.: G01N 27/414

(54) **KAPAZITIV KONTROLLIERTER FELDEFFEKT-GASSENSOR, ENTHALTEND EINE HYDROPHOBE SCHICHT**
CAPACITIVELY CONTROLLED FIELD EFFECT GAS SENSOR COMPRISING A HYDROPHOBIC LAYER
DETECTEUR DE GAZ A EFFET DE CHAMP COMMANDE CAPACITIVEMENT COMPRENANT UNE COUCHE HYDROPHOBE

(30) Priorität: 30.07.2003 DE 10335163
(43) Veröffentlichungstag der Anmeldung: 26.04.2006
(73) Patentinhaber: Micronas GmbH, 79108 Freiburg i. Br. (DE); Albert-Ludwigs-Universität Freiburg, 79104 Freiburg (DE)
(72) Erfinder: RÜHE, Jürgen, 79656 Eichstetten (DE); SAMUEL, J.D.Jeyaprakash, S., Milpitas, CA 95035 (US); FRERICHS, Heinz-Peter, 79271 St.Peter (DE); LEHMANN, Mirko, 79117 Freiburg (DE)
(74) Vertreter: Koch Müller Patentanwaltsgesellschaft mbH
(86) Internationale Anmeldenummer: PCT/EP2004/008309
(87) Internationale Veröffentlichungsnummer: WO 2005/012896

(56) Entgegenhaltungen:
- EP-A- 0 947 829
- DE-A- 10 118 367
- DE-A- 10 161 214
- US-A- 5 900 128
- PATENT ABSTRACTS OF JAPAN Bd. 0113, Nr. 73 (P-643), 5. Dezember 1987 (1987-12-05) & JP 62 144062 A (FUJI ELECTRIC CO LTD), 27. Juni 1987 (1987-06-27)
- BURGMAIR M ET AL: "Contribution of the gate insulator surface to work function measurements with a gas sensitive FET" PROCEEDINGS OF IEEE SENSORS 2002. ORLANDO, FL, JUNE 12 - 14, 2002, IEEE INTERNATIONAL CONFERENCE ON SENSORS, NEW YORK, NY : IEEE, US, Bd. VOL. 1 OF 2. CONF. 1, 12. Juni 2002 (2002-06-12), Seiten 439-442, XP010605133 ISBN: 0-7803-7454-1
- PATENT ABSTRACTS OF JAPAN Bd. 0133, Nr. 14 (P-899), 18. Juli 1989 (1989-07-18) & JP 01 083147 A (OLYMPUS OPTICAL CO LTD), 28. März 1989 (1989-03-28)
- PATENT ABSTRACTS OF JAPAN Bd. 0062, Nr. 62 (P-164), 21. Dezember 1982 (1982-12-21) & JP 57 156553 A (TOKYO SHIBAURA DENKI KK), 27. September 1982 (1982-09-27)
- PRUCKER O ET AL: "Photochemical attachment of polymer films to solid surfaces via monolayers of benzophenone derivatives" JOURNAL OF THE AMERICAN CHEMICAL SOCIETY 29 SEP 1999 UNITED STATES, Bd. 121, Nr. 38, 29. September 1999 (1999-09-29), Seiten 8766-8770, XP002307406 ISSN: 0002-7863 in der Anmeldung erwähnt
- GERGINTSCHEW Z ET AL: "The capacitively controlled field effect transistor (CCFET) as a new low power gas sensor" SENSORS AND ACTUATORS B, ELSEVIER SEQUOIA S.A., LAUSANNE, CH, Bd. 36, Nr. 1, Oktober 1996 (1996-10), Seiten 285-289, XP004061082 ISSN: 0925-4005
- POHL R ET AL: "Realization of a new sensor concept: emproved ccfet and sgfet type gas sensors in hybrid flip-chip technology" TRANSDUCERS '03, Bd. 1, 9. Juni 2003 (2003-06-09), Seiten 135-138, XP010646487

## Beschreibung

Die Erfindung betrifft einen Gassensor nach dem Oberbegriff von Anspruch 1.

Ein derartiger Gassensor ist aus DE 43 33 875 C2 bekannt. Er weist eine gassensitive Schicht auf, die mit einer Änderung ihrer Austrittsarbeit auf Gaseinwirkungen reagiert. Auf dem Kanalbereich des Gassensors ist eine elektrisch isolierende Schicht angeordnet, die das Substrat, den Source- und den Drain-Bereich überdeckt. Zwischen dieser Kanalisolierung und der gassensitive Schicht ist ein Luftspalt gebildet. Dies entspricht dem Prinzip des Suspended Gate Feldeffekttransitsors (SGFET). Die durch die Anwesenheit des Gases in der gassensitiven Schicht induzierte elektrische Spannung koppelt kapazitiv über den Luftspalt an die Kanaloberfläche und induziert Ladungen in die Struktur des SGFET. Der Kanalbereich wird von einer Guardelektrode umrahmt, welche den Kanalbereich gegen elektrische Potentiale, die außerhalb des von der Guardelektrode umgrenzten Oberflächenbereichs des Gassensors angeordnet sind, abschirmt. Der Gassensor hat jedoch den Nachteil, dass das Messsignal des SGFET außer von der zu messenden Konzentration des Gases auch von dem elektrischen Widerstand zwischen Guardring und Kanalbereich, der durch Feuchte beeinflusst wird, abhängig ist. Ungünstig ist dabei vor allem, dass die Messgenauigkeit des Gassensors bei langsamen Gas-Konzentrationsänderungen mit zunehmendem Feuchtigkeitsgehalt überproportional abnimmt.

Aus DE 101 18 367 C2 ist auch bereits ein verbesserter Gassensor bekannt, bei dem zwischen dem Guardring und dem Kanalbereich eine Oberflächenprofilierung ausgebildet ist, die Erhöhungen und Vertiefungen aufweist. Durch diese relativ einfach und kostengünstig zu realisierende Maßnahme wird die Weglänge zwischen dem Guardring und dem Kanalbereich vergrößert und somit der an der Oberfläche zwischen dem Guardring und dem Kanalbereich fließende Faradayische Strom entsprechend reduziert. Obwohl sich dieser Gassensor in der Praxis in einer Vielzahl von Anwendungen bewährt hat, weist er dennoch Nachteile auf So nimmt auch bei diesem Gassensor bei langsamen Gas-Konzentrationsänderungen die Messgenauigkeit mit zunehmendem Feuchtigkeitsgehalt überproportional ab.

Aus EP 1 191 332 A1 ist ferner ein Gassensor der eingangs genannten Art bekannt, der im selben Feldeffekttransistor neben der gassensitiven Schicht zusätzlich auch eine feuchtesensitive Schicht aufweist, die nach dem gleichen Messprinzip auslösbar ist wie die gassensitive Schicht. Nach Angabe der Offenlegungsschrift soll es dadurch möglich sein, bei bekannter Temperatur Feuchteeinflüsse im Vergleich zu der zu messenden Gasreaktion zu bestimmen und durch Heranziehung des Feuchtemesssignals bei dem Gassensor die Querempfindlichkeit auf Feuchte zu reduzieren. Nachteilig ist dabei jedoch, dass außer dem zusätzlichem Feuchtesensor auch noch eine aufwendige und teuere Kompensationsschaltung benötigt wird. Ungünstig ist außerdem, dass eine derartige Kompensation jeweils nur für eine bestimmte Temperatur gültig ist, so dass bei Temperaturschwankungen auch noch ein Temperaturmesssignal erfasst und berücksichtigt werden muss.

Es besteht deshalb die Aufgabe, einen Gassensor der eingangs genannten Art zu schaffen, der bei einem einfachen und kompakten Aufbau eine hohe Messgenauigkeit ermöglicht Dabei soll die Messgenauigkeit weitgehend unabhängig von Feuchteeinflüssen sein.

Diese Aufgabe wird mit den Merkmalen des Anspruchs 1 gelöst.

In vorteilhafter Weise wird durch diese überraschend einfache Lösung die Adsorption von Feuchtigkeit an der Oberfläche des Gassensors erschwert oder sogar vollständig verhindert. Dadurch wird der Transport von Ionen zwischen dem Kanalbereich bzw. der Sensorelektrode und davon beabstandeten Stellen des Gassensors, die ein anderes elektrische Potential aufweisen als der Kanalbereich bzw. die Sensorelektrode, vor allem bei einem hohen Feuchtigkeitsgehalt des Gases erheblich eingeschränkt. Somit bleibt die Messgenauigkeit des Gassensors weitgehend konstant, wenn sich der Feuchtigkeitsgehalt in dem Gas verändert. Außerdem wird eine hohe Langzeitstabilität der Messgenauigkeit ermöglicht. Die hydrophobe Schicht ist erfindungsgemäβ auf einer elektrisch isolierenden Schicht angeordnet.

Die hydrophobe Schicht ist bevorzugt als ultrahydrophobe Schicht ausgebildet.

In Burgmair M et al. "Contribution of the gate insulatior surface to work function measurements with a gas sensitive FET" ist zwar bereits ein Gassensor gemäß dem Oberbegriff von Anspruch 1 bekannt, wobei an der Oberfläche des Gassensors eine hydrophobe Passivierungsschicht (Si₃N₄) angeordnet ist, die sich zwischen der gassensitiven Schicht und dem Kanalbereich befindet. Der Kontaktwinkel der Passivierungsschicht beträgt jedoch nur etwa 60°. Die Passivierungsschicht wird deshalb relativ leicht mit Wasser benetzt. Der Gassensor hat daher eine entsprechend größere Querempfindlichkeit auf Feuchtigkeit. Auch bei dem in DE 01 61 214 A1 und Pohl R. et al "Realization of a new sensor concept improved ccfet and sgfet gas sensors in hybrid flip-chip technology" beschriebenen Feldeffekttransistoren wird als Passivierungsschicht eine Si₃N-₄-Schicht verwendet.

Aus US 5 900 128 ist ferner ein Feldeffekttransistor bekannt, der eine Oberflächenbeschichtung aus Polyimid aufweist. Auch der Kontaktwinkel dieser Schicht ist wesentlich kleiner als 90°.

Bei einer bevorzugten Ausführungsform der Erfindung weist der Gassensor an seiner Oberfläche einen elektrisch leitfähigen Guardring auf, der den Kanalbereich und/oder die Sensorelektrode mit Abstand zu dem Kanalbereich und/oder der Sensorelektrode umgrenzt, wobei die hydrophobe Schicht zumindest in einem zwischen dem Guardring und dem Kanalbereich und/oder der Sensorelektrode befindlichen Bereich der Oberfläche des Gassensors angeordnet ist. Durch den Guardring wird verhindert, dass das Potential über dem Kanalbereich bzw. das Potential der Sensorelektrode nach einer bestimmten Zeit durch die an der Oberfläche des Gassensors noch vorhandene Leitfähigkeit auf das Potential des an den Kanalbereich kapazitiv angekoppelten Pols der gasempfindlichen Schicht oder das Potential des Guardringes gezogen wird. Somit wird eine Potenfialdrift vermieden und es wird eine noch größere Messgenauigkeit erreicht.

Bei eine zweckmäβigen nicht erfindungs gemäβen der Erfindung erstreckt sich die hydrophobe Schicht durchgängig über den Kanalbereich und/oder die Sensorelektrode. Der Gassensor ist dann besonders einfach und kostengünstig herstellbar, da die hydrophobe Schicht ganzflächig auf die Oberfläche des Gassensors aufgebracht und somit ein Maskierungsschritt eingespart werden kann.

Vorteilhaft ist, wenn die hydrophobe Schicht von dem Kanalbereich und/oder der Sensorelektrode beabstandet ist und den Kanalbereich und/oder die Sensorelektrode vorzugsweise ring- oder rahmenförmig umgrenzt. Dadurch kann bei einer hydrophoben Schicht, in der bei einem Kontakt mit einem Störgas, das sich von dem Gas, für das die gassensitive Schicht empfindlich ist, unterscheidendet, eine elektrische Störspannung induziert wird, der Einfluss dieser Störspannung auf das Messsignal und somit die Querempfindlichkeit des Gassensors für das Störgas reduziert werden.

Bei der erfindungsgemäβen Ausführungsform der Erfindung beträgt der mit Wasser gemessene, auf ein plane Oberfläche bezogene statische Kontaktwinkel der hydrophoben Schicht mindestens 90°, insbesondere mindestens 105° und bevorzugt mindestens 120° beträgt. Vor allem bei einem Kontaktwinkel von mindestens 120° kann eine besonders hohe und weitgehend vom Feuchtigkeitsgehalt des Gases unabhängige Messgenauigkeit des Gassensors erreicht werden. Der Kontaktwinkel kann mit bekannten Standardmessverfahren bei Raumtemperatur bestimmt werden.

Bei einer zweckmäßigen Ausgestaltung der Erfindung sind Moleküle der hydrophoben Schicht kovalent an die Oberfläche einer daran angrenzenden elecktrisch isolierenden Schicht des Gassensors gebunden. Dadurch ist es möglich, bei der Fertigung des Gassensor die hydrophobe Schicht direkt an der daran angrenzenden Schicht des Gassensors zu befestigen.

Vorteilhaft ist, wenn die hydrophobe Schicht mindestens ein Polymer enthält. Die hydrophobe Schicht kann dann bei der Fertigung des Gassensors bei Raumtemperatur auf die Oberfläche des Gassensors aufgebracht werden, wodurch die bereits auf dem Substrat befindlichen Implanfierungsbereich und Strukturen thermisch geschont werden.

Besonders vorteilhaft ist, wenn das Polymer ein fluoriertes und bevorzugt ein perfluoriertes Polymer ist. Durch die in diesen Polymeren enthalten stark elektronegativen CF-Gruppen kann auch bei einem hohen Feuchteanteil in dem zu messenden Gas, beispielsweise bei einer relativen Feuchte von 90%, noch eine hohe Messgenauigkeit bei der Messung der Gaskonzentration erreicht werden.

Bei einer anderen vorteilhaften Ausführungsform der Erfindung ist das Polymer über eine vorzugsweise als Monolage ausgebildete Zwischenschicht mit einer benachbarten, elektrisch isolierenden Schicht des Gassensors verbunden, wobei die Zwischenschicht mindestens eine an der benachbarten Schicht verankerte reaktive Gruppe aufweist, und wobei das Polymer vorzugsweise über eine kovalente Bindung an die Zwischenschicht gekoppelt ist. Dabei ist es sogar möglich, das hydrophobe Polymer bei der Herstellung des Gassensors zunächst ganzflächig auf die Zwischenschicht aufzubringen und dann unter Einwirkung von mittels einer Schattenmaske auf die Oberfläche des Gassensors projizierter optischer Strahlung nur in bestimmten Teilbereichen der Oberfläche des Gassensors photochemisch an die Zwischenschicht zu binden. In den übrigen Teilbereichen kann das hydrophobe Polymer danach beispielsweise durch Abwaschen von der Oberfläche des Gassensors entfernt werden. Insgesamt ergibt sich somit ein Gassensor mit einer strukturierten, nur an bestimmten Stellen seiner Oberfläche angeordneten hydrophoben Schicht.

Vorteilhaft ist, wenn die hydrophobe Schicht eine Oberflächenprofilierung mit Vorsprüngen und Vertiefungen aufweist Dadurch kann eine noch größere Messgenauigkeit erreicht werden.

Die Vertiefungen sind bevorzugt als Gräben oder Nuten ausgebildet, die rahmen- oder ringförmig um den Kanalbereich und/oder die Sensorelektrode umlaufen.

Nachfolgend sind Ausführungsbeispiel der Erfindung anhand der Zeichnung näher erläutert. Es zeigt zum Teil stärker schematisiert:
- Fig. 1: einen Längsschnitt durch einen Gassensor, der unter einer strichliniert dargestellten gasempfindlichen Schicht einen ISFET aufweist
- Fig. 2: einen Querschnitt durch den in Fig. 1 gezeigten Gassensor entlang der in Fig. 1 mit II bezeichneten Schnittlinie,
- Fig. 3: einen Längsschnitt durch einen Gassensor, der unter einer strichliniert dargestellten gasempfindlichen Schicht einen CCFET aufweist,
- Fig. 4: einen Querschnitt durch den in Fig. 3 gezeigten Gassensor entlang der in Fig. 3 mit IV bezeichneten Schnittlinie,
- Fig. 5: eine schematische Darstellung der photochemischen Bindung eines hydrophoben Polymers an eine auf einer elektrischen Isolationsschicht immobilisierten Schicht mit Linker-Molekülen.

Ein im Ganzen mit 1 bezeichneter Gassensor weist ein Substrat 2 eines ersten Ladungsträgertyps auf, das z.B. aus p-dotiertem Silizium bestehen kann. Auf dem Substrat 2 sind eine Drain 3 und eine Source 4 eines zweiten Ladungsträgertyps angeordnet. Die Drain 3 und die Source 4 können beispielsweise aus n-dotiertem Silizium bestehen. Die Drain 3 ist über in der Zeichnung nur teilweise dargestellte elektrische Leiterbahnen mit einem Drain-Anschluss 5 verbunden. In entsprechender Weise ist die Source 4 mit einem Source-Anschluss 6 verbunden. Der Drain-Anschluss 5 und der Source-Anschluss 6 sind jeweils an einer auf dem Substrat 2 deponierten Schicht 7 angeordnet.

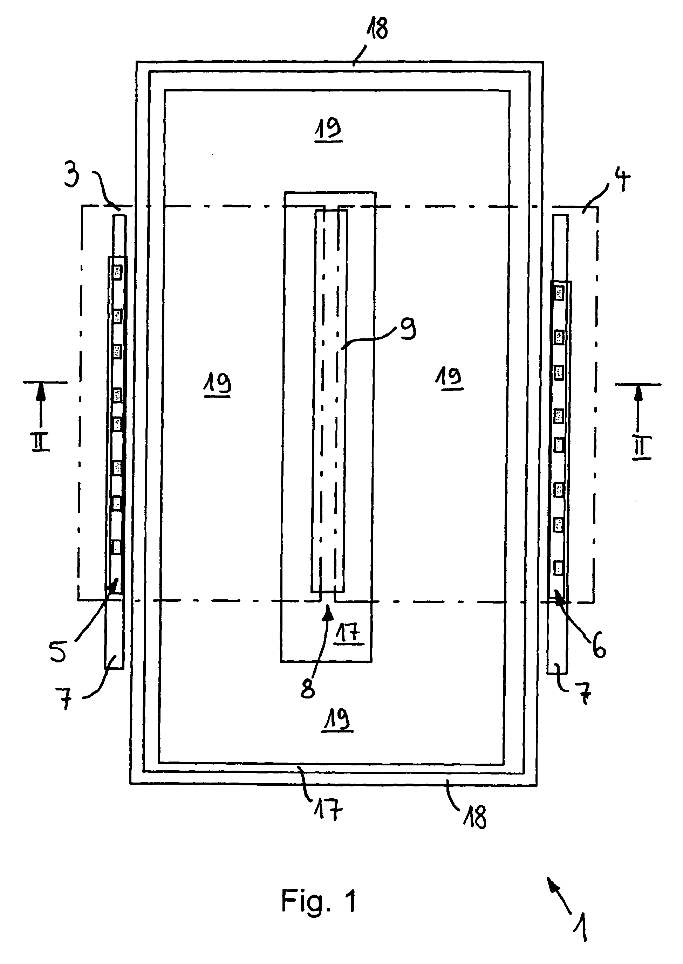

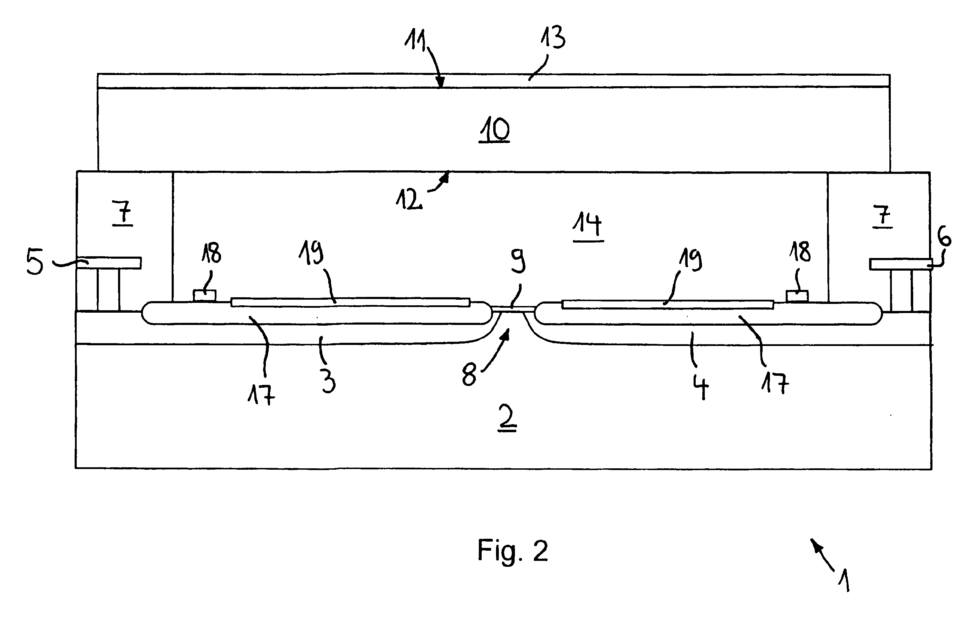

Zwischen Drain 3 und Source 4 ist in dem Substrat 2 ein Kanalbereich 8 gebildet, auf dem bei dem Ausführungsbeispiel nach Fig. 1 und 2 eine elektrisch isolierende Dünnoxidschicht 9 angeordnet ist, die als Gatedielektrikum dient. Die Dünnoxidschicht 9 hat etwa eine Schichtdicke von 3-150 nm.

Wie in Fig. 2 besonders gut erkennbar ist, weist der Gassensor 1 ferner eine gassensitiven Schicht 10 auf, die an ihren einander abgewandten Flachseiten Pole 11, 12 hat, zwischen denen in Abhängigkeit von der Konzentration eines mit der Schicht 10 in Kontakt befindlichen Gases eine gasinduzierte elektrische Spannung auftritt. Zur Detektion der Spannung ist die gassensitive Schicht 10 mit ihrem einen Pol 12 über einen Luftspalt 14 kapazitiv an den Kanalbereich 8 gekoppelt. Der andere Pol 11 ist mit einer Gegenelektrode 13 verbunden, an der ein elektrisches Bezugspotential anliegt. Der Luftspalt 14 weist einen Zugang zu dem zu detektierenden Gas auf und ist zwischen deponierten Schichten 7, auf denen die gassensitive Schicht 10 abgestützt ist.

Bei dem Ausführungsbeispiel nach Fig. 1 und 2 ist der Kanalbereich 8 offen ausgebildet (ISFET) und über das Dünnschichtoxid und den Luftspalt 14 direkt an die gassensitive Schicht 10 kapazitiv gekoppelt. Deutlich ist erkennbar, dass der Kanalbereich 8 an der der gassensitiven Schicht 10 gegenüberliegenden Seite des Luftspalts 14 angeordnet ist.

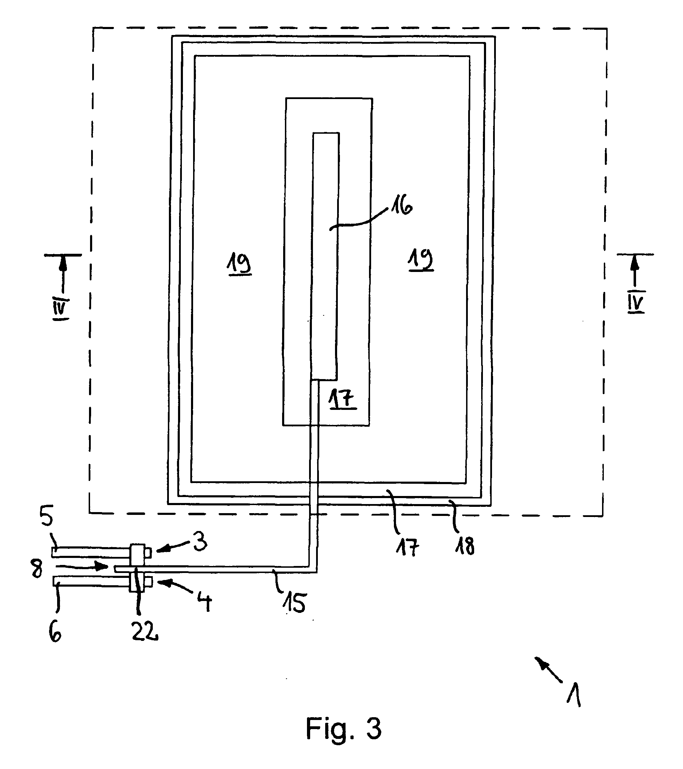

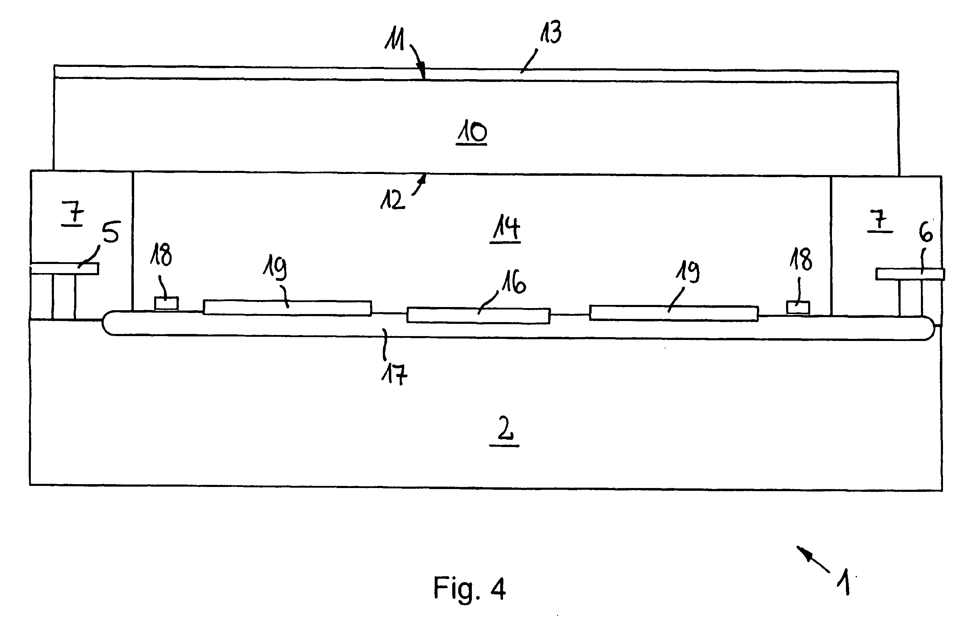

Bei dem Ausführungsbeispiel nach Fig. 3 und 4 ist der Kanalbereich 8 seitlich neben der gassensitiven Schicht 10 in dem Substrat 2 angeordnet und mit einer Gateelektrode 22 abgedeckt. Zur kapazitiven Ankopplung des Kanalbereichs 8 an die gassensitive Schicht 10 ist die Gateelektrode 22 über eine Leiterbahn 15 mit einer Sensorelektrode 16 verbunden, die an der dem Pol 12 der gassensitiven Schicht 10 gegenüberliegenden Seite des Luftspalts 14 auf einer auf dem Substrat 2 befindlichen elektrischen Isolationsschicht 17 angeordnet ist. Die Isolationsschicht 17 kann beispielsweise eine SiO₂-Schicht sein.

Der Gassensor 1 weist an seiner Oberfläche außerdem einen elektrisch leitfähigen Guardring 18 auf, der bei dem Ausführungsbeispiel nach Fig. 1 und 2 den Kanalbereich 8 und bei dem Ausführungsbeispiel nach Fig. 3 und 4 die zu dem zu dem Kanalbereich 8 führende Sensorelektrode 16 umgrenzt. Dabei ist bei dem Ausführungsbeispiel nach Fig. 1 und 2 zwischen dem Guardring 18 und dem Kanalbereich 8 und bei dem Ausführungsbeispiel nach Fig. 3 und 4 zwischen dem Guardring 18 und der Sensorelektrode 16 jeweils ein Abstand vorgesehen. Um den Kanalbereich 8 gegen außerhalb des von dem Guardring 18 umgrenzten Oberflächenbereichs des Gassensors 2 befindliche elektrische Potentiale abzuschirmen, liegt der Guardring 18 auf einem definierten elektrischen Potential.

Bei dem Ausführungsbeispiel nach Fig. 1 und 2 ist zwischen dem Guardring 18 und dem Kanalbereich 8 an der Oberfläche des Gassensors 1 eine hydrophobe Schicht 19 angeordnet. Diese befindet sich auf einer elektrischen Isolationsschicht 17, die auf der Drain 3, der Source 4 und außerhalb des Kanalbereichs 8 befindlichen Bereichen des Substrat 2 angeordnet ist. In Fig. 1 ist erkennbar, dass die hydrophobe Schicht 19 den Kanalbereich 8 rahmenförmig umgrenzt und mit Abstand zu dem Kanalbereich 8 und dem Guardring 18 endet. Durch die hydrophobe Schicht 19 wird in dem zwischen dem Guardring 18 und dem Kanalbereich 8 befindlichen Teil der Oberfläche des Gassensors die Adsorption von in dem Gas befindlichem Wasser erheblich erschwert. Dadurch werden ein hoher elektrischer Widerstand an der Oberfläche und ein hohe Messgenauigkeit des Gassensors ermöglicht.

Bei dem Ausführungsbeispiel nach Fig. 3 und 4 ist die hydrophobe Schicht 19 zwischen dem Guardring 18 und der Sensorelektrode 16 auf der Isolationsschicht 17 angeordnet. In Fig. 4 ist erkennbar, dass die hydrophobe Schicht 19 die Sensorelektrode 16 rahmenförmig umgrenzt und mit Abstand zu der Sensorelektrode 16 und dem Guardring 18 endet. Durch die hydrophobe Schicht 19 wird in dem zwischen dem Guardring 18 und der Sensorelektrode 16 befindlichen Teil der Oberfläche des Gassensors 1 die Adsorption von in dem Gas befindlichem Wasser erschwert.

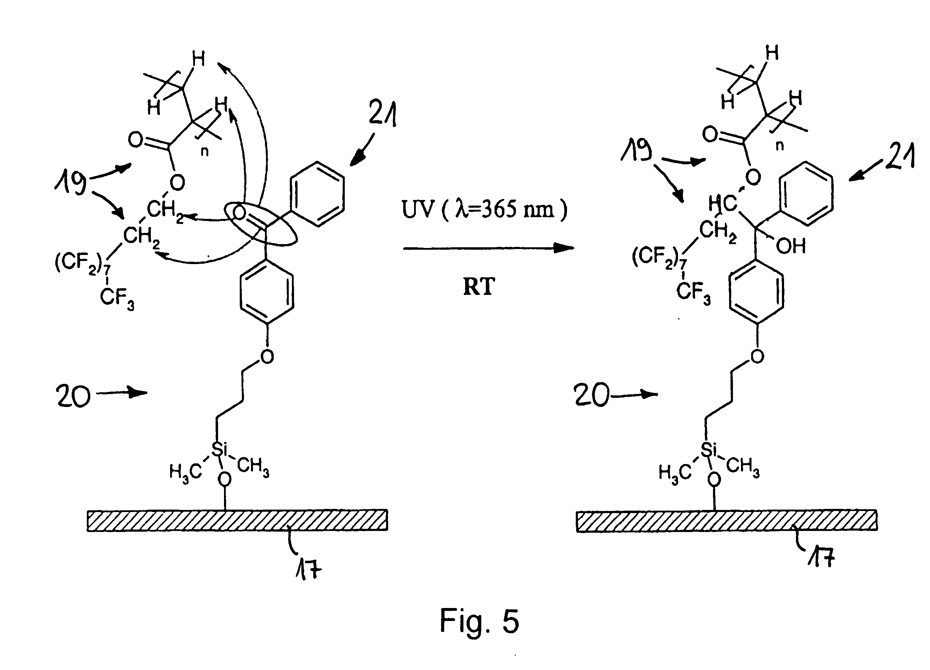

Die hydrophobe Schicht besteht aus einem Polymer, vorzugsweise aus Poly(heptadecafluroacrylat). Bei der Fertigung des Gassensors 1 wird die hydrophobe Schicht 19 über eine Zwischenschicht 20 an der Isolationsschicht 17 befestigt. Dazu wird zunächst die Zwischenschicht 20 in Form einer Monolage eines benzophenon-funktionalisierten Monochlorosilans auf der Isolationsschicht 17 aufgebracht. In Fig. 5 ist erkennbar, dass in der Zwischenschicht 20 während der UV-Belichtung freie Radikale entstehen, die beim Kontaktieren der Isolationsschicht 17 an diese anbinden und dadurch die Zwischenschicht 20 an der Isolationsschicht 17 befestigen.

Danach wird eine dünne Lage Poly(heptadecafluroacrylat) ganzflächig auf der Zwischenschicht 20 deponiert. Nun werden mit Hilfe einer Schattenmaske die Stellen, an denen später die hydrophobe Schicht 19 sein soll, mit UV-Strahlung bestrahlt In Fig.. 5 ist erkennbar, dass die Zwischenschicht 20 eine photoreaktive Benzophenon-Gruppe 21 aufweist, die bei Bestrahlung mit UV-Licht an ein benachbartes Polymer der späteren hydrophoben Schicht 19 bindet. Dabei übernimmt die Benzophenon-Gruppe 21 von dem benachbarten Polymer ein Wasserstoffatom, derart, dass zwischen der Benzophenon-Gruppe 21 und dem benachbarte Polymer eine kovalente Verknüpfung gebildet wird (vgl. Prucker, O., Rühe, J. et al., Photochemical Attachment of Polymer Films to Solid Surfaces via Monolayers of Benzophenone Derivates, J. Am. Chem. Soc. (1999), 121, Seite 8766-8770). Nachdem das Polymer der hydrophoben Schicht 19 auf diese Weise in bestimmten Bereichen an der Oberfläche der Isolationsschicht 17 gebunden wurde, werden die ungebunden, an den nicht belichteten Stellen der Oberfläche befindlichen Polymere zur Bildung der strukturierten hydrophoben Schicht 19 entfernt, beispielsweise indem sie mit einem Lösungsmittel abgewaschen werden.

Erwähnt werden soll noch, dass auch andere nicht erfindungsgemäβe Ausführungsbeispiele möglich sind, bei denen sich die hydrophobe Schicht 19 unterbrechungsfrei über den Kanalbereich 8, die Sensorelektrode 16 und/oder den Guardring 18 erstrecken kann. Bei der Herstellung eines solchen Gassensors 1 kann die hydrophobe Schicht 19 auch direkt auf der Isolationsschicht 17 deponiert werden. Dies kann in der Weise geschehen, dass das hydrophobe Trichloro(1H, 1H,2H,2H-perflurooctyl)silan (TPFS) aus der gasförmigen Phase bei einer Temperatur von etwa 100°C auf der Isolationsschicht 17 abgeschieden werden. Das Abscheiden des TPFS erfolgt vorzugsweise unter Abwesenheit von Feuchtigkeit, damit Querverbindungen und Inhomogenitäten in dem auf der Oberfläche abgeschiednen TPFS-Film vermieden werden. Außerdem ist darauf zu achten, dass während des Abscheidungsprozesses keine Staubpartikel an der Oberfläche anhaften.

## Patentansprüche

1. Gassensor (1) mit einem Substrat (2) eines ersten Ladungsträgertyps, auf dem eine Drain (3) und eine Source (4) eines zweiten Ladungsträgertyps angeordnet sind, wobei zwischen Drain (3) und Source (4) ein Kanalbereich (8) gebildet ist, und mit einer gassensitiven Schicht (10), die Pole (11, 12) hat, zwischen denen in Abhängigkeit von der Konzentration eines mit der Schicht (10) in Kontakt befindlichen Gases eine gasinduzierte Spannung auftritt, wobei die gas-sensitive Schicht (10) zur Messung der Spannung mit ihrem einen Pol (12) über einen Luftspalt (14) kapazitiv an den Kanalbereich (8) gekoppelt ist und mit dem anderen Pol (11) mit einer ein Bezugspotential aufweisenden Gegenelektrode (13) verbunden ist,
**dadurch gekennzeichnet, dass**
an der Oberfläche des Gassensors (1) eine hydrophobe Schicht (19) deren mit Wasser gemessener, auf eine plane Oberfläche bezogene statische Kontaktwinkel mindestens 90° beträgt, und die hydrophobe Schicht (19) bereichsweise auf einer elektrischen Isolationsschicht (17) angeordnet ist und (a) die hydrophobe Schicht (19) den Kanal-bereich (8) ring-oder rahmenförmig umgrenzt, wobei die Isolationsschicht auf der Drain (3), der Source (4) und außerhalb des Kanalbereichs (8) befindlichen Bereichen des Substrats (2) angeordnet ist, oder (b) die hydrophobe Schicht (19) eine Sensorelektrode (16) ring-oder rahmenförmig umgrenzt, wobei die Sensorelektrode (16) auf der sich auf dem Substrat (2) befindlichen Isolationsschicht (17) angeordnet ist und mit einer an den Kanalbereich (8) angeordneten Gateelektrode (22) elektrisch verbunden ist.

2. Gassensor (1) nach Anspruch 1, **dadurch gekennzeichnet, dass** er an seiner Oberfläche einen elektrisch leitfähigen Guardring (18) aufweist, der den Kanalberelch (8) und/oder die zu dem zu dem Kanalbereich (8) führende Sensorelektrode (16) mit Abstand zu dem Kanalbereich (8) und/oder der Sensorelektrode (16) umgrenzt, und dass die hydrophobe Schicht (19) zumindest in einem zwischen dem Guardring (18) und dem Kanalbereich (8) und/oder der Sensorelektrode (16) befindlichen Bereich der Oberfläche des Gassensors (1) angeordnet ist.

3. Gassensor (1) nach einem der Ansprüche 1 bis 2, **dadurch gekennzeichnet, dass** die hydrophobe Schicht (19) von dem Kanalbereich (8) und/oder der Sensorelektrode (8) beabstandet ist

4. Gassensor (1) nach einem der Ansprüche 1 bis 3, **dadurch gekennzeichnet, dass** der mit Wasser gemessene, auf ein plane Oberfläche bezogene statische Kontaktwinkel der hydrophoben Schicht (19) mindestens 105° und bevorzugt mindestens 120° beträgt.

5. Gassensor (1) nach einem der Ansprüche 1 bis 4, **dadurch gekennzeichnet, dass** Moleküle der hydrophoben Schicht (19) kovalent an die Oberfläche einer daran angrenzenden, vorzugsweise halbleitenden oder elektrisch isolierenden Schicht des Gassensors (1) gebunden.

6. Gassensor (1) nach einem der Ansprüche 1 bis 5, **dadurch gekennzeichnet dass** die hydrophobe Schicht (19) mindestens ein Polymer enthält.

7. Gassensor (1) nach Anspruch 6, **dadurch gekennzeichnet, dass** das Polymer ein fluoriertes und bevorzugt ein perfluoriertes Polymer ist.

8. Gassensor (1) nach Anspruch 6 oder 7, **dadurch gekennzeichnet, dass** das Polymer über eine vorzugsweise als Monolage ausgebildete Zwischenschicht (20) mit der benachbarten elektrisch isolierenden Schicht (17) des Gassensors (1) verbunden ist, dass die Zwischenschicht (20) mindestens eine an der benachbarten Schicht verankerte reaktive Gruppe aufweist, und dass das Polymer vorzugsweise über eine kovalente Bindung an die Zwischenschicht (20) gekoppelt ist

9. Gassensor (1) nach einem der Ansprüche 1 bis 8, **dadurch gekennzeichnet, dass** die hydrophobe Schicht (19) eine Oberflächenprofilierung mit Vorsprüngen und Vertiefungen aufweist.

10. Gassensor (1) nach einem der Ansprüche 1 bis 9, **dadurch gekennzeichnet, dass** die Vertiefungen als Gräben oder Nuten ausgebildet sind, die vorzugsweise rahmen- oder ringförmig um den Kanalbereich (8) und/oder die Sensorelektrode (16) umlaufen.

## Claims

1. Gas sensor (1) with a substrate (2) of a first charge carrier type, on which a drain (3) and a source (4) of a second charge carrier type are arranged, wherein a channel region (8) is formed between drain (3) and source (4), and with a gas-sensitive layer (10) having poles (11, 12) between which a gas-induced voltage arises in dependence on the concentration of a gas disposed in contact with the layer (10), wherein the gas-sensitive layer (10) is for measurement of the voltage capacitively coupled by one pole (12) thereof with the channel region (8) by way of an air gap (14) and is connected by the other pole (11) thereof with a counter-electrode (13) having a reference potential, **characterised in that** arranged on the surface of the gas sensor (1) is a hydrophobic layer (19), the static contact angle - which is measured by water and refers to a planar surface - of which is at least 90°, and the hydrophobic layer (19) is arranged regionally on an electrical insulation layer (17) and (a) the hydrophobic layer (19) delimits the channel region (8) annularly or in the manner of a frame, wherein the insulation layer is arranged on the drain (3), the source (4) and the regions of the substrate (2) disposed outside the channel region (8), or (b) the hydrophobic layer (19) delimits a sensor electrode (16) annularly or in frame-like manner, wherein the sensor electrode (16) is arranged on the insulation layer (17), which is disposed on the substrate (2), and is electrically connected with a gate electrode (22) arranged at the channel region (8).

2. Gas sensor (1) according to claim 1, **characterised in that** it has at its surface an electrically conductive guard ring (18) which delimits the channel region (8) and/or the sensor electrode (16), which leads to the channel region (8), at a spacing from the channel region (8) and/or the sensor electrode (16) and that the hyrdophobic layer (19) is arranged at least in a region of the surface of the gas sensor (1) disposed between the guard ring (18) and the channel region (8) and/or the sensor electrode (16).

3. Gas sensor (1) according to one of claims 1 and 2, **characterised in that** the hydrophobic layer (19) is spaced from the channel region (8) and/or the sensor electrode (16).

4. Gas sensor (1) according to any one of claims 1 to 3, **characterised in that** the static contact angle, which is measured by water and refers to a planar surface, of the hydrophobic layer (19) is at least 105° and preferably at least 120°.

5. Gas sensor (1) according to any one of claims 1 to 4, **characterised in that** molecules of the hydrophobic layer (19) are covalently bound to the surface of a preferably semiconductive or electrically insulating layer, which adjoins thereat, of the gas sensor (1).

6. Gas sensor (1) according to any one of claims 1 to 5, **characterised in that** the hydrophobic layer (19) contains at least one polymer.

7. Gas sensor (1) according to any one of claims 1 to 6, **characterised in that** the polymer is a fluorinated and preferably a perfluorinated polymer.

8. Gas sensor (1) according to claim 6 or 7, **characterised in that** the polymer is connected with the adjacent electrically insulating layer (17) of the gas sensor (1) by way of an intermediate layer (20) preferably constructed as a mono-layer, that the intermediate layer (20) has at least one reactive group anchored at the adjacent layer and that the polymer is coupled to the intermediate layer (20) preferably by way of a covalent bond.

9. Gas sensor (1) according to any one of claims 1 to 8, **characterised in that** the hydrophobic layer (19) has a surface profiling with projections and recesses.

10. Gas sensor (1) according to any one of claims 1 to 9, **characterised in that** the recesses are formed as trenches or grooves which preferably encircle the channel region (8) and/or the sensor electrode (6) in frame-like manner or annularly.

## Revendications

1. Détecteur de gaz (1) avec un substrat (2) d'un premier type de porteurs de charge, sur lequel un drain (3) et une source (4), d'un deuxième type de porteurs de charge, sont disposés, une zone de canal (8) étant formée entre le drain (3) et la source (4), et une couche (10) sensible aux gaz, comprenant des pôles (11, 12), entre lesquels se produit une tension induite par les gaz, en fonction de la concentration d'un gaz se trouvant en contact avec la couche (10), la couche (10) sensible aux gaz étant couplée capacitivement à la zone de canal (8), avec l'un de ses pôles (12), par l'intermédiaire d'un entrefer (14), pour la mesure de la tension, et étant reliée à l'autre pôle (11) à une contre-électrode (13) présentant un potentiel de référence,
**caractérisé en ce que**,
sur la surface du détecteur de gaz (1), est disposée une couche (19) hydrophobe, dont l'angle de contact statique, se référant à une surface plane, hydro-mesuré a une valeur d'au moins 90°, et la couche (19) hydrophobe est disposée, au moins par zones, sur une couche d'isolation électrique (17) et (a) la couche (19) hydrophobe entoure en anneau ou encadre la zone de canal (8), la couche d'isolation étant disposée sur le drain (3), la source (4) et des zones, se trouvant à l'extérieur de la zone de canal (8) du substrat (2), ou (b) la couche (19) hydrophobe entoure en anneau ou encadre une électrode de capteur (16), l'électrode de capteur (16) étant disposée sur la couche d'isolation (17) se trouvant sur le substrat (2) et étant reliée électriquement à une électrode de grille (22) disposée sur la zone de canal (8).

2. Détecteur de gaz (1) selon la revendication 1, **caractérisé en ce qu'**il présente sur sa surface un anneau de garde (18) conducteur de l'électricité, circonscrivant la zone de canal (8) et/ou l'électrode de capteur (16) menant à la zone de canal (8), à distance de la zone de canal (8) et/ou de l'électrode de capteur (16), et **en ce que** la couche (19) hydrophobe est disposée au moins dans une zone, se trouvant entre l'anneau de garde (18) et la zone de canal (8) et/ou l'électrode de capteur (16), de la surface du détecteur de gaz (1).

3. Détecteur de gaz (1) selon l'une des revendications 1 à 2, **caractérisé en ce que** la couche (19) hydrophobe est espacée de la zone de canal (8) et/ou de l'électrode de capteur (16).

4. Détecteur de gaz (1) selon l'une des revendications 1 à 3, **caractérisé en ce que** l'angle de contact statique, se référant à une surface plane, hydro-mesuré de la couche (19) hydrophobe a une valeur d'au moins 105° et, de préférence, d'au moins 120°.

5. Détecteur de gaz (1) selon l'une des revendications 1 à 4, **caractérisé en ce que** des molécules de la couche (19) hydrophobe sont liées de manière covalente à la surface d'une couche lui étant limitrophe, de préférence semi-conductrice ou électriquement isolante, du détecteur de gaz (1).

6. Détecteur de gaz (1) selon l'une des revendications 1 à 5, **caractérisé en ce que** la couche (19) hydrophobe contient au moins un polymère.

7. Détecteur de gaz (1) selon la revendication 6, **caractérisé en ce que** le polymère est un polymère fluoré et, de préférence, perfluoré.

8. Détecteur de gaz (1) selon la revendication 6 ou 7, **caractérisé en ce que** le polymère est relié, par l'intermédiaire d'une couche intermédiaire (20), réalisée de préférence sous forme de monocouche, à la couche (17) voisine, isolante électriquement, du détecteur de gaz (1), **en ce que** la couche intermédiaire (20) présente au moins un groupe réactif, ancré à la couche voisine, et **en ce que** le polymère est couplé à la couche intermédiaire (20), de préférence par une liaison covalente.

9. Détecteur de gaz (1) selon l'une des revendications 1 à 8, **caractérisé en ce que** la couche (19) hydrophobe présente un profilage de surface avec des saillies et des évidements.

10. Détecteur de gaz (1) selon l'une des revendications 1 à 9, **caractérisé en ce que** les évidements sont réalisés sous la forme de tranchées ou de rainures, de préférence en forme de cadre ou d'anneau, entourant la zone de canal (8) et/ou l'électrode de capteur (16).
